# EUROPÄISCHE PATENTSCHRIFT

(11) **EP 0 312 873 B1**
(45) Veröffentlichungstag und Bekanntmachung des Hinweises auf die Patenterteilung: **24.11.1993**
(21) Anmeldenummer: 88116780.3
(22) Anmeldetag: 10.10.1988
(51) Int. Cl.: C07C 275/26, C08G 18/78, C08G 18/80

(54) **N,N'-Bis(trans-4-isocyanatocyclo-hexyl)harnstoff, Verfahren zu dessen Herstellung und Verwendung**
N,N'-bis(trans-4-isocyanato-cyclohexyl)urea, process for its preparation and use
N,N'-bis(trans-4-isocyanato-cyclohexyl)urée, procédé de préparation et utilisation

(30) Priorität: 20.10.1987 DE 3735469
(43) Veröffentlichungstag der Anmeldung: 26.04.1989
(73) Patentinhaber: BAYER AG, 51368 Leverkusen (DE)
(72) Erfinder: Ruckes, Andreas, Dr., D-5090 Leverkusen 3 (DE); Rasshofer, Werner, Dr., D-5000 Köln 80 (DE); Kopp, Richard, Dr., D-5000 Köln 80 (DE); Grögler, Gerhard, Dr., D-5090 Leverkusen 1 (DE)

(56) Entgegenhaltungen:
- Keine Entgegenhaltungen.

## Beschreibung

Die vorliegende Erfindung betrifft N,N′-Bis(trans-4-isocyanatocyclohexyl)harnstoff, erhalten aus der Umsetzung von Cyclohexan-1,4-diisocyanat (CHDI) mit einem hohen Transisomerengehalt und Wasser in einem inerten Lösungsmittel. Der erfindungsgemäße Harnstoff, der gegebenenfalls einen kleinen Anteil an oligomeren Harnstoffen enthält, läßt sich vorteilhaft beim Aufbau von Polyurethanen verwenden.

Es ist bekannt, daß Monoisocyanate mit Wasser unter Bildung N-substituierter Carbamidsäurederivate reagieren, die unter Kohlendioxidabspaltung und Reaktion mit weiterem Isocyanat in Harnstoffderivate übergehen. Analog erhält man aus Diisocyanaten und Wasser durch Polyaddition Polyharnstoffe.

Es ist ferner bekannt, daß man bei Einhaltung bestimmter Reaktionsbedingungen niedermolekulare Harnstoffdiisocyanate der allgemeinen Formel 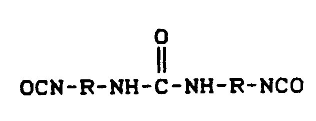
isolieren kann, wenn man 1 Mol Wasser mit mindestens 2 Mol eines Diisocyanates bei niedrigen Temperaturen zwischen 0° C und 30° C in einem Lösungsmittel reagieren läßt. Wie in der DAS-1 101 394 beschrieben, sind für diese Reaktion nur solche Diisocyanate geeignet, die wie z.B. das 2,4-Toluylendiisocyanat Isocyanatgruppen mit verschieden großer Reaktionsfreudigkeit aufweisen. Aliphatische Diisocyanate sind wegen ihrer geringen Reaktionsfähigkeit gegenüber Wasser wenig geeignet.

Obwohl also Harnstoffdiisocyanate generell beschrieben sind, sind bislang konkrete Beschreibungen des Diisocyanatoharnstoffes auf der Basis von CHDI nicht bekannt.

Im Einklang mit dem Stand der Technik erreicht man beim Versuch handelsübliches CHDI unter den üblichen Bedingungen mit Wasser in einem inerten Lösungsmittel umzusetzen, selbst unter Anwendung basischer Katalyse und langer Reaktionszeiten nur geringe Mengen eines polymeren Harnstoffes mit niedrigem NCO-Gehalt.

In Übereinstimmung damit steht auch die im Rahmen von kinetischen Untersuchungen von S.W. Wong und K.C. Frisch in Adv. in Urethan Sci. and Technol. 8 (1981) auf den S. 75-92 beschriebene Harnstoffbildung in stark verdünnten Lösungen von trans-CHDI mit Wasser in Cellosolveacetat bei 50° C, die mit einer Halbwertszeit von 3623 min zum Polyharnstoff mit einem NCO-Gehalt gegen 0 % führt.

Überraschenderweise wurde nun gefunden, daß man N,N′-Bis(4-isocyanatocyclohexyl)harnstoff, der gegebenenfalls nur einen kleinen Anteil an oligomeren Harnstoffen enthält, in guter Ausbeute erhält, wenn man Cyclohexan-1,4-diisocyanat mit einem hohen Transisomerengehalt bei niedrigen Temperaturen (ca. 40° C) in inerten Lösungsmitteln, in einer Konzentration ≧ 10 Gew.-%, bezogen auf das verwendete Lösungsmittel, unter Anwendung basischer Katalysatoren mit Wasser im Molverhältnis 2:1 umsetzt.

Der erfindungsgemäße Harnstoff läßt sich vorteilhaft zum Aufbau von Polyurethan-Elastomeren verwenden, die neben hervorragender Wärmestabilität ein ausgezeichnetes mechanisches Werteniveau besitzen.

Ein weiterer Vorteil besteht darin, daß die ungünstigen Eigenschaften des Ausgangsisocyanates, den durch den geringen Dampfdruck bedingten stechenden Grund bzw. die tränenreizende Wirkung, beim entsprechenden Harnstoff nicht auftreten, wodurch eine einfache und sichere Verarbeitbarkeit gewährleistet ist.

Gegenstand der vorliegenden Erfindung ist somit der N,N'(4-isocyanatocyclohexyl)harnstoff I, der gegebenenfalls einen kleinen Anteil an oligomeren Harnstoffen enthält, erhältlich aus der Umsetzung von 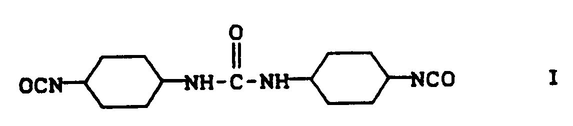
Cyclohexan-1,4-diisocyanat mit einem hohen Transisomerengehalt und Wasser unter den im folgenden näher beschriebenen Bedingungen.

Das Cyclohexan-1,4-diisocyanat, das zur Herstellung der erfindungsgemäßen Harnstoffes eingesetzt wird, hat einen hohen Gehalt an Transisomeren, dessen Anteil, bezogen auf das CHDI, mindestens etwa 80 Gew.-%, bevorzugt 90 Gew.-% beträgt. Günstig ist es, wenn der Anteil mindestens 95 Gew.-%, besonders bevorzugt mindestens 99 Gew.-% beträgt. Vorteilhaft ist es, wenn das CHDI im wesentlichen aus den Transisomeren besteht, das heißt, der Anteil des cis-Isomeren ist gleich Null oder kann praktisch vernachlässigt werden.

Als Ausgangskomponente für das erfindungsgemäße Verfahren ist z.B. ein trans-Cyclohexan-1,4-diisocyanat geeignet, das nach dem in der DE-PS 2 710 595 näher beschriebenen Verfahren hergestellt wird.

Als Lösungsmittel werden solche verwendet, in dem das CHDI und wenigstens ein Teil des benötigten Wassers löslich sind. Es soll weiterhin keine NCO-reaktiven Gruppen enthalten, also inert oder nicht-reaktiv gegenüber Isocyanaten sein.

Ether, wie Diethyl- oder Diisopropylether, Ketone, wie Aceton, Methylethylketon und Ester, wie Isopropylacetat oder Cellosolveacetat stellen geeignete Lösungsmittel dar. Halogenkohlenwasserstoffe, in denen Wasser praktisch unlöslich ist, sind z.B. ungeeignet als erfindungsgemäße Lösungsmittel. Es ist jedoch nicht notwendig, daß sich die gesamte benötigte Menge Wasser im verwendeten Lösungsmittel löst. Bevorzugte Lösungsmittel sind Diisopropylether, Methylethylketon und Cellosolveacetat.

Die Konzentration des CHDI im Lösungsmittel ist im allgemeinen unkritisch. Im Falle zu großer Verdünnung wird jedoch der Harnstoff unter Umständen mit stark erniedrigtem NCO-Gehalt erhalten.

Bevorzugt wird eine Konzentration von ≧ 10 Gew.-%, insbesondere ≧ 30 Gew.-% CHDI, bezogen auf das Lösungsmittel, eingehalten. Bei zu stark konzentrierten Lösungen besteht andererseits die Gefahr, daß das ausfallende Reaktionsprodukt mit unreagiertem CHDI verunreinigt ist.

Die Menge an Wasser entspricht in etwa der theoretisch benötigten Menge zur Reaktion mit zwei Isocyanat-Gruppen. Wird zu wenig Wasser verwendet, bleibt unreagiertes Ausgangsisocyanat zurück, welches abgetrennt werden muß. Andererseits wird bei Verwendung von zuviel Wasser nur ein Polyharnstoff mit stark erniedrigtem NCO-Gehalt erhalten.

Der entstehende Harnstoff ist in den oben erwähnten Lösungsmitteln nahezu unlöslich und entzieht sich so einer Weiterreaktion der noch vorhandenen NCO-Gruppen mit Wasser, so daß im allgemeinen von dem eingesetzten CHDI nur eine NCO-Gruppe pro Molekül an der Harnstoffgruppenbildung teilnimmt.

Die Reaktionstemperatur sollte im allgemeinen unterhalb 55° C gehalten werden. Bei höheren Reaktionstemperaturen steigt die Löslichkeit des Harnstoffes an, so daß eine Weiterreaktion mit Wasser über Bildung von polymeren oder oligomeren Harnstoffen erfolgen kann.

Zur Erzielung befriedigender Raum-Zeit-Ausbeuten muß zur Beschleunigung der Reaktion zwischen Wasser und dem reaktionsträgen aliphatischen CHDI ein Katalysator eingesetzt werden. Zur Anwendung kommen bevorzugt die in der Polyurethanchemie üblicherweise eingesetzten Katalysatoren, wie z.B. tertiäre Amine (N,N-Dimethylbenzylamin, Triethylamin, Diazobicyclooktan) oder metallorganische Verbindungen (Tributylzinnacetat, Di-n-butylzinndiacetat, Zinn(II)dioctoat, Dibutylzinndilaurat).

Die Katalysatormenge wird vorzugsweise so gewählt, daß die entwickelte Reaktionswärme das Gemisch nicht über 40° C erwärmt, so daß eine externe Kühlung entfallen kann.

Nach Beendigung der Reaktion wird das ausgefallene Produkt über ein geeignetes Filter abgesaugt und mit einem inerten Lösungsmittel, wie z.B. Aceton oder Petrolether gewaschen und in einem Trockenschrank, vorzugsweise in Vakuum, bei niedrigen Temperaturen getrocknet.

Der NCO-Gehalt des nach diesem Verfahren erhaltenen Produktes liegt normalerweise nur geringfügig unterhalb des berechneten NCO-Gehaltes. Um befriedigende Raum-Zeit-Ausbeuten zu erhalten, müssen jedoch die Reaktionsbedingungen im allgemeinen so gewählt werden, daß Produkte mit einem gegenüber der Theorie verringertem NCO-Gehalt, bei denen also geringfügige Oligoharnstoffbildung eingetreten ist, erhalten werden. Es zeigt sich nämlich, daß alle Maßnahmen, die zu einer Erhöhung der Reaktionsgeschwindigkeit führen, wie erhöhte Temperatur, erhöhte Katalysatormenge, polare, wasserlösliche Lösungsmittel, zwar eine Steigerung der Ausbeute bei verringerter Reaktionszeit, aber gleichzeitig immer eine Verringerung des NCO-Gehaltes des erfindungsgemäßen Harnstoffes bewirken, Die Produkte lassen sich jedoch verwenden, wenn die Oligoharnstoffbildung bestimmte Grenzen nicht überschreitet.

Das lagerstabile und leicht handhabbare Produkt fällt in Form eines feinteiligen, kristallinen Pulvers in Ausbeuten, abhänggig von den Reaktionsbedingungen, bis zu 85 % und NCO-Gehalten von mindestens 21 Gew.-% an.

Der erfindungsgemäße Harnstoff läßt sich in seiner niedermolekularen bzw. niederoligomeren Form mit Vorteil zum Aufbau von Polyurethansystemen verwenden.

Eine bevorzugte Anwendung beinhaltet die Herstellung von Gießelastomeren, in denen als NCO-reaktive Komponenten mit aromatischen Amingruppen terminierte Polyether bzw. besonders bevorzugt Polyester eingesetzt werden, die vorzugsweise nach der EP-AS 0 219 035 durch Hydrolyse von endständige Isocyanatgruppen aufweisende Verbindungen erhalten werden. Bei diesem Verfahren werden insbesondere zwei oder drei Hydroxygruppen aufweisende Polyether bzw. Polyester zu NCO-Prepolymeren umgesetzt und in einem zweiten Schritt die Isocyanatgruppen durch Hydrolyse in eine Aminogruppe überführt. Man erhält Elastomere mit hervorragendem Wärmestand und ausgezeichneten mechanischen Eigenschaften.

Die folgenden Beispiele erläutern die Herstellung des erfindungsgemäßen Harnstoffes und seine Anwendung zur Herstellung von hochwertigen Polyurethanelastomeren.

Für die erfindungsgemäßen Beispiele wurde, wenn nicht anders erwähnt, trans-Cyclohexan-1,4-diisocyanat der Firma Akzo ("Elate 166"®) eingesetzt.

### Beispiel 1

830 g (5 Mol) Elate-CHDI werden in 2.000 g Methylethylketon (MEK) gelöst. Nach Zugabe einer Lösung von 46 g (2,56 Mol) Wasser in 250 ml MEK/Aceton 1:1 und 0,9 g N,N-Dimethylbenzylamin (DB) wird die Reaktionsmischung 60 Stunden bei Raumtemperatur gerührt. Das ausgefallene Produkt wird abgesaugt, erst mit Aceton und anschließend mit Petrolether gewaschen und im Vakuumtrockenschrank von den Lösungsmitteln befreit. Das Produkt resultiert in Form eines feinteiligen, kristallinen Pulvers in einer Ausbeute von 666 g (87 %), bezogen auf eingesetztes Elate-CHDI, mit einem NCO-Gehalt von 22,9 Gew.-%.

| | | | | |
|---|---|---|---|---|
| C: | ber. | 58,8 | gef. | 58,4 |
| H: | ber. | 7,2 | gef. | 7,7 |
| N: | ber. | 18,3 | gef. | 18,5 |

### Beispiel 2

166 g (1 Mol) Elate-CHDI werden in 480 g Diisopropylether (DPE) gelöst. Nach Zugabe einer Lösung von 9,8 g (0,51 Mol) Wasser in 80 g Aceton und 0,21 g N,N-Dimethylbenzylamin (DB) wird die Reaktionsmischung 7 Tage bei Raumtemperaturen gerührt. Das ausgefallene Produkt wirdd abgesaugt, mit Aceton gewaschen und im Vakuumtrockenschrank von Lösungsmittel befreit. Man erhält 43 g (28 %) des Produktes mit einem NCO-Gehalt von 26,6 Gew.-%.

| | | | | |
|---|---|---|---|---|
| C: | ber. | 58,8 | gef. | 58,4 |
| H: | ber. | 7,2 | gef. | 7,6 |
| N: | ber. | 18,3 | gef. | 18,4 |

### Beispiel 3

83 g (0,5 Mol) "Elate"-CHDI werden in 250 ml Ethylenglykolethyletheracetat (EGA) gelöst. Nach Zugabe einer Lösung von 4,6 g (0,256 Mol) Wasser in 25 ml EGA/Aceton 1:1 und 0,09 g DB wird die Reaktionsmischung 19 Stunden bei Raumtemperatur gerührt. Das ausgefallene Produkt wird abgesaugt, mit Aceton gewaschen und im Vakuumtrockenschrank von Lösungsmittel befreit. Man erhält 10 g (13 %) des gewünschten Produktes mit einem NCO-Gehalt von 27,1 Gew.-%.

### Beispiel 4

83 g (0,5 Mol) "Elate"-CHDI werden in 250 ml EGA gelöst. Nach Zugabe einer Lösung von 4,6 g (0,256 Mol) Wasser in 25 ml EGA/Aceton 1:1 und 1,87 g Diaza-2,2,2-bicyclooktan (DABCO) wird die Reaktionsmischung 19 Stunden bei Raumtemperatur gerührt. Die Aufarbeitung erfolgt wie in Beispiel 2. Man erhält 65 g (85 %) des gewünschten Produktes mit einem NCO-Gehalt von 22,0 Gew.-%.

### Beispiel 5

### Vergleichsbeispiel - nicht erfindungsgemäß - nicht - stöchiometrische Wassermenge in hoher Verdünnung

9,71 g (0,058 Mol) "Elate"-CHDI werden in 250 ml EGA gelöst. Nach Zugabe einer Lösung von 1,08 g (0,06 Mol) Wasser in 250 ml EGA und 0,22 g DABCO wird die Reaktionsmischung 19 Stunden bei 50° C gerührt. Innerhalb dieser Zeit konnte kein Ausfall eines Produktes verzeichnet werden.

### Beispiel 6

### Vergleichsbeispiel - nicht erfindungsgemäß wie 5 nur in höherer Konzentration

40,4 g (0,24 Mol) "Elate"-CHDI werden in 125 ml EGA gelöst. Nach Zugabe einer Lösung von 4,5 g (0,25 Mol) Wasser in 125 ml EGA und 0,93 g DABCO wird die Reaktionsmischung 19 Stunden bei 50° C gerührt. Die Aufarbeitung erfolgt wie in Beispiel 3. Man erhält 15 g (40,8 %) eines polymeren Harnstoffes, der keine nachweisbaren NCO-Gruppen mehr enthält.

### Beispiel 7

### wie 5, mit der stöchiometrisch richtigen Menge Wasser

19,4 g (0,116 Mol) "Elate"-CHDI werden in 500 ml EGA gelöst. Nach Zugabe einer Lösung von 1,08 g (0,06 Mol) Wasser in 500 ml EGA und 0,44 g DABCO wird die Reaktionsmischung 60 Stunden bei 50° C gerührt. Die Aufarbeitung erfolgt wie in Beispiel 3. Man erhält 4,7 g (26,5 %) eines Harnstoffes mit einem NCO-Gehalt von 14,6 Gew.-%.

### Beispiel 8

### Vergleichsbeispiel - nicht erfindungsgemäß

166 g (1 Mol) eines CHDI, das zu 37, 2 Gew.-% aus cis-Isomeren und zu 62,8 Gew.-% aus dem trans-Isomeren besteht, werden in 400 g MEK gelöst. Nach Zugabe einer Lösung von 9,2 g (0,51 Mol) Wasser in 50 ml MEK/Aceton 1:1 und 0,18 g DB wird die Reaktionsmischung für 60 Stunden bei Raumtemperatur gerührt. Die Aufarbeitung erfolgt wie in Beispiel 1. Man erhält 110 g (71,9 %) eines oligomeren Harnstoffes mit einem NCO-Gehalt von 8,7 Gew.-%.

### Beispiel 9

### Vergleichsbeispiel - nicht erfindungsgemäß

166 g (1 Mol) eines CHDI, das zu 83 Gew.-% aus cis-Isomeren und zu 16 Gew.-% aus dem trans-Isomeren besteht, werden in 800 g MEK gelöst. Nach Zugabe einer Lösung von 9,2 g (0,51 Mol) Wasser in 50 ml Aceton und 0,18 g DB wird für 19 Stunden bei Raumtemperatur gerührt. Da kein Ausfall eines Produktes erkennbar war, werden weitere 24 Stunden bei 50° C gerührt. Erst nach Zusatz von insgesamt 4,5 g DB und weiteren 60 Stunden Reaktionszeit wurde nach bekannter Aufarbeitung 73 g (47,7 %) eines Harnstoffes mit einem NCO-Gehalt von 16,0 Gew.-% erhalten.

### Beispiel 10

### Beispiel zur Herstellung eines Polyurethan-Elastomeren nach der Gießelastomer-Technik

Der in diesem Beispiel verwendete Polyester mit endständigen aromatisch gebundenen Aminogruppen wurde gemäß EP-AS 0 219 035 durch Hydrolyse eines NCO-Prepolymeren mit einem NCO-Gehalt von 3,66 %, hergestellt aus einen Polyester auf der Basis Adipinsäure, Ethylenglykol mit der OH-Zahl 56 und 2,4-Diisocyanatotoluol im Äquivalentverhältnis NCO:OH = 2:1, erhalten.

200 g des obigen Aminopolyesters werden bei 50-60° C mit 34,7 g CHDI aus Beispiel 1 in Form eines gemahlenen Pulvers (Teilchengröße; 10-30 µm) vermischt, gut homogenisiert und im Wasserstrahlvakuum entgast. Man erhält ein Reaktivsystem mit einer Topfzeit bei 50-60° C von mehreren Stunden, ohne daß es zu einer unerwünschten Viskositätserhöhung in diesem Zeitraum kommt.

Das gut gießbare Reaktivsystem wird in eine auf 80-100° C vorgewärmte und mit Trennmittel versehene Form gegossen, die anschließend auf 110-150° C erhitzt wird. Nach der Verfestigung des Ansatzes (1-2 Stunden) wird das Formteil entfernt und ca. 2 Stunden bei 120° C getempert. Man erhält einen hochwertigen PUR-Elastomeren mit einer hervorragenden Wärmestabilität und dem unten angegebenen mechanischen Wertniveau.

| | | |
|---|---|---|
| Zugfestigkeit | (DIN 53504) | 28,7 MPa |
| Bruchdehnung | (DIN 53504) | 500 % |
| Weiterreißwiderstand | (DIN 53515) | 80,5 KN/m |
| Shore-Härte A | (DIN 53505) | 97 |
| Elastizität | (DIN 53512) | 40 % |

## Patentansprüche

1. N,N'-Bis(trans-4-isocyanatocyclohexyl)harnstoff.

2. N,N'-Bis(4-isocyanatocyclohexyl)harnstoff mit einem Anteil des trans-Isomeren von mindestens 80 Gew.-%.

3. Vefahren zur Herstellung von N,N'-Bis(4-isocyanatocyclohexyl)harnstoff mit einem Anteil des trans-Isomeren von mindestens 80 Gew.-%, dadurch gekennzeichnet, daß man Cyclohexan-1,4-diisocyanat mit einem Transisomerengehalt von mindestens 80 Gew.-%, bevorzugt mindestens 90 Gew.-%, mit einem Mol Wasser je 2 Mol Diisocyanat in einem inerten Lösungsmittel hydrolysiert.

4. Verwendung der Harnstoffe nach Anspruch 1 oder 2 als Reaktionskomponente von NCO-reaktiven Verbindungen zur Herstellung von Polyurethanen.

5. Verwendung nach **Anspruch 4** dadurch gekennzeichnet, daß als NCO-reaktive Verbindungen mit aromatischen Aminogruppen terminierte Polyether bzw. insbesondere Polyester eingesetzt werden.

6. Verwendung nach **Anspruch 5** dadurch gekennzeichnet, daß die Aminopolyester durch Hydrolyse von endständige Isocyanatgruppen aufweisenden Polyesterprepolymeren erhalten werden.

## Claims

1. N,N'-Bis-(trans-4-isocyanatocyclohexyl)-urea.

2. N,N'-bis(4-isocyanatohexyl)urea containing at least 80% by weight of the trans isomer.

3. A process for the preparation of N,N'-bis-(4-isocyanatocyclohexyl)-urea containing at least 80% by weight of the trans isomer, characterised in that cyclohexane-1,4-diisocyanate having a trans-isomer content of at least 80% by weight, preferably at least 90% by weight, is hydrolysed with 1 mol of water for every 2 mol of isocyanate in an inert solvent.

4. Use of the ureas according to Claim 1 or 2 as reaction components for isocyanate reactive compounds for the preparation of polyurethanes.

5. Use according to Claim 4, characterised in that the isocyanate reactive compounds used are polyethers or in particular polyesters which are terminated with aromatic amino groups.

6. Use according to Claim 5, characterised in that the amino polyesters are obtained by the hydrolysis of polyester prepolymers containing terminal isocyanate groups.

## Revendications

1. N,N'-bis(trans-4-isocyanatocyclohexyl)urée.

2. N,N'-bis(4-isocyanatocyclohexyl)urée ayant une proportion de l'isomère trans d'au moins 80 % en poids.

3. Procédé de production de N,N'-bis(4-isocyanatocyclohexyl)urée ayant une proportion d'isomère trans d'au moins 80 % en poids, caractérisé en ce qu'on hydrolyse du cyclohexane-1,4-diisocyanate ayant une teneur en isomère trans d'au moins 80 % en poids, de préférence d'au moins 90 % en poids, avec une mole d'eau pour 2 moles de diisocyanate dans un solvant inerte.

4. Utilisation des urées suivant la revendication 1 ou 2 comme composant réactionnel de composés réactifs vis-à-vis de groupes NCO pour la production de polyuréthannes.

5. Utilisation suivant la revendication 4, caractérisée en ce qu'on utilise comme composés réactifs vis-à-vis de groupes NCO des polyéthers ou en particulier des polyesters terminés par des groupes amino aromatiques.

6. Utilisation suivant la revendication 5, caractérisée en ce que les aminopolyesters sont obtenus par hydrolyse de prépolymères de polyesters portant des groupes isocyanate terminaux.
